# EUROPEAN PATENT SPECIFICATION

(11) **EP 0 384 672 B1**
(45) Date of publication and mention of the grant of the patent: **09.08.1995**
(21) Application number: 90301728.3
(22) Date of filing: 16.02.1990
(51) Int. Cl.: A61B 17/32

(54) **Ultrasonic surgical scalpel**
Chirurgisches Ultraschallskalpell
Scalpel ultrasonique pour la chirurgie

(30) Priority: 18.02.1989 JP 18167/89
(43) Date of publication of application: 29.08.1990
(73) Proprietor: Takase, Haruo, Tokyo (JP)
(72) Inventor: Takase, Haruo, Tokyo (JP)
(74) Representative: Watkins, David

(56) References cited:
- EP-A- 0 282 684
- CH-A- 481 638
- FR-A- 2 302 713
- FR-A- 2 474 857

## Description

This invention relates to an ultrasonic surgical scalpel capable of effectively shattering subcutaneous or organic tissues of a living body into its mushy state by use of ultrasonic vibrations and sucking out the shattered tissues by use of sucking force.

There has been well known an ultrasonic surgical scalpel for use in a plastic surgical operation to remove subcutaneous adipose tissues or an organic surgery to remove liver or other organic tissues and so on, which comprises an ultrasonic vibrator incorporated in a handpiece and a hollow horn connected to the vibrator. Vibrations generated by the vibrator are magnified in amplitude in a matter of two times to several ten times when propagating from the basal end attached to the vibrator to the leading end of the horn.

When performing the plastic surgical operation to remove, for example, subcutaneous adipose tissues of a patient's body, the leading end of the aforesaid scalpel horn is inserted into under the skin of the patient's body, and then, the vibrator is driven to vibrate the horn so as to shatter the subcutaneous adipose tissues into its mushy state. While shattering the subcutaneous tissues with ultrasonic vibrations generated by the vibrator, sucking force is applied to inside the hollow horn to suck out the subcutaneous tissues shattered into the mushy state as noted above through a suction passage in the horn. Thus, the subcutaneous tissues can be shattered with the ultrasonic vibrations and removed out of the patient's body through the horn.

In the prior art ultrasonic scalpel, the hollow horn is used as a suction tube through which the shattered tissues or fat in the mushy state are sucked out and provided in its leading end with a suction mouth. In a general way, this suction mouth is open just frontward in the axial (lengthwise) direction of the horn. That is, the secant plane defining the mouth at the leading end of the horn perpendicularly intersects the axis of the horn. This conventional ultrasonic scalpel has however entailed a disadvantage that it produces relatively large resistance when being inserted into the subcutaneous or organic tissues so much as to advance against the tissues with difficulty because the secant plane forming the mouth is perpendicular to the axis of the horn. This is one of the reasons why the surgical operation is difficult. Besides, forcible insertion of the horn into the subcutaneous or organic tissues causes blood vessels, nerve tissues and so on to be injured needlessly, consequently to elongate the period for healing an operation wound.

Particularly in a case of inserting the scalpel horn into between the skin and the subcutaneous tissue of the living body, the scalpel horn cannot be moved forward along the inner surface of the skin in a desired direction and is apt to advance into the subcutaneous tissue, because the suction mouth is open straight frontward in the axial direction of the horn.

Furthermore, the conventional ultrasonic scalpel is generally connected to an external aspirator in order to aspirate subcutaneous tissues shattered into its mushy state with ultrasonic vibrations. Generally, sucking force generated by the aspirator is adjusted by operating a suction adjusting means provided on the aspirator or a suction regulating valve mounted on the handpiece. Either way, the work becomes onerous where the adjusting means or regulating valve is frequently operated to control the sucking force to be given to inside the scalpel horn in the midst of a surgical operation. Also, the surgical scalpel with the latter suction regulating valve is complicated in structure and awkward.

CH-A-481,638 describes an ultrasonic surgical instrument having the constructional features of the preamble of Claim 1.

One object of this invention is to provide an ultrasonic surgical scalpel totally free from such defects of the conventional ultrasonic scalpels mentioned above, which can effectively shatter and suck out unnecessary subcutaneous or organic tissues to be removed without needlessly injuring other tissues such as blood vessels and nerve tissues in advancing the scalpel in under the tissues.

Another object of the invention is to provide an ultrasonic surgical scalpel having a suction regulating means which is simple in structure and can be readily operated with the finger tip of an operator.

The invention provides an ultrasonic surgical scalpel in accordance with Claim 1.

According to this invention, the leading end can be formed acute or substantially round because the suction mouth need not to be formed so as to be open just frontward. That is, the suction mouth can be formed simply by diagonally cutting the leading end part of the horn or in the circumferential surface of the horn. As a result, the leading end of the horn may be formed aslant or round so as to enable the horn to be readily and smoothly inserted into under the skin, organic tissues or the like without needlessly injuring blood vessels, nerve tissues and so on.

With the suction regulating mouth formed in the handpiece so as to communicate with the suction passage inside the horn, sucking force which is generated by an external aspirator connected to the scalpel and given to inside the horn can be easily regulated with the finger tip of an operator as it is held with the operator's hand.

One way of carrying out the invention is described in detail below with reference to the accompanying drawings which illustrate only specific embodiments, in which:-

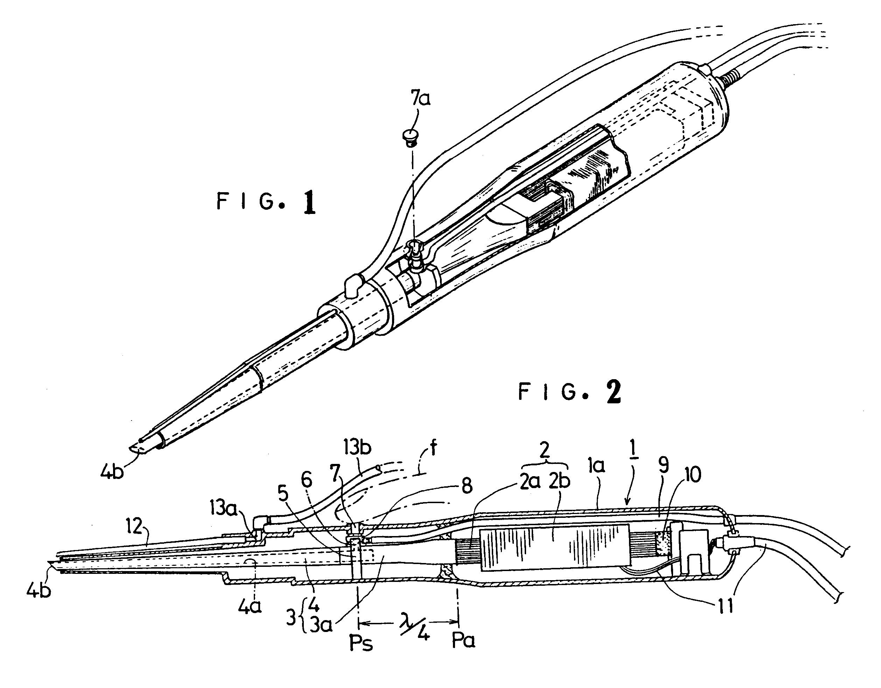

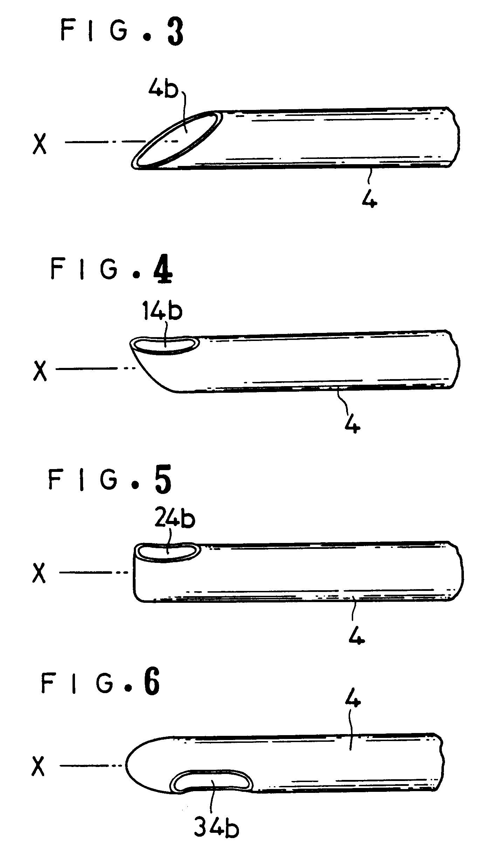

Figure 1 is a partially section perspective view showing a first embodiment of the ultrasonic surgical scalpel according to this invention; Figure 2 is a side view in section showing the scalpel of Figure 1; Figure 3 is an enlarged side view of the leading end part of the same scalpel; Figure 4 is a partly enlarged side view of another embodiment of the invention; Figure 5 is a partly enlarged side view of still another embodiment of the invention; and Figure 6 is a partly enlarged side view of a further embodiment of the invention.

The first preferred embodiment of the ultrasonic surgical scalpel according to this invention will be described with reference to Figures 1 and 2. In the drawings, reference numeral 1 denotes a handpiece and 1a a casing. The casing 1a accommodates an ultrasonic vibrator 2 and a horn 3 connected to the vibrator 2. The substantially front half part of the horn 3 serves as a suction nozzle 4 having a suction passage 4a bored along the axis of the horn. In this embodiment, the suction nozzle 4 is separately formed and detachably attached to the substantially rear half part (basal half) of the horn 4, but it may of course be incorporated with the basal half of the horn in one body.

The leading end part of the suction nozzle 4 somewhat protrudes forwards from the leading end of the casing 1. In the illustrated embodiment, the suction nozzle 4 is provided in the leading end part thereof with a suction mouth 4b.

The ultrasonic vibrator 2 is composed of a ferromagnetic core 2a and an electromagnetic coil 2b wound around the core 2a. By applying an alternating current to the coil 2b, electrostrictive vibrations are generated in the core 2a.
Otherwise, there may be used a magnetostrictive vibrator which generates vibrations by utilization of magnetostrictive phenomenon. However, the vibrator of this type is feeble in electromagnetic converting efficiency (about 30%), namely, about 70% of electric energy applied to the vibrator in the form of electricity becomes extinct in the form of heat. This implies the need of a cooling system for refluxing air or water around the vibrator. Therefore, if the magnetostrictive vibrator is employed in the ultrasonic scalpel, such a cooling system should be incorporated therein.

To be more specific, the electrostrictive vibrator used preferably in the scalpel of the invention has an outstanding advantage that it can generate vibrations with a high efficiency of about 90% or above, and further, stably produce an sufficient energy even when load applied thereto is fluctuated due to its high energy modulus. By this reason, the scalpel according to this invention employs the electrostrictive vibrator.

The horn 3 incorporating the suction nozzle 4 has a function of magnifying the amplitude of vibrations generated by the vibrator and effectively shattering the subcutaneous tissue of a living body into its mushy state with the vibrations transmitted to its leading end being brought into contact with the tissue. Thus, the horn 3 may preferably be made of metallic material having large endurance limit so as to sufficiently withstand vibrations having the increased amplitude (about 100»m) of high frequency. As an example, a titanium alloy which is relatively small in metallic fatigue is suitable for the horn.

The horn 3 is supported by a supporting member 5 made of elastic material at a point (supporting point Ps) substantially one-fourth the wavelength of vibration λ away from the connection point (Pa) at which the horn 3 and the vibrator 2 are connected, i.e. λ/4.

At the supporting point Ps, there is formed a connection port 6 communicating with the suction passage 4a formed inside the suction nozzle 4. To the connection port 6 is connected a suction regulating mouth 7 formed in the casing 1a of the handpiece 1 through the medium of a connection member 8. This connection member 8 is also connected to a suction pipe 9 which extends outside through the rear end of the casing 1a and is connected with an external aspirator (not shown).

In other words, the suction regulating mouth 7 is substantially formed in the course of the suction pipe 9 connected to the connection port 6 formed in the rear end part of the suction nozzle 4, namely, in the substantially central part of the horn, and may practically be formed in the casing 1a at a position based on human engineering so as to be readily opened and closed directly with the finger tip of an operator. In a case where the suction regulating mouth 7 need not be opened, it may be closed detachably with a lid member 7a.

Besides the requirement that the length from the supporting point Ps at which the supporting member 5 is disposed to the connection portion Pa should be substantially λ/4 as noted above, it is desirable that the length from the supporting point Ps to the leading end of the horn 3 (suction nozzle 4) be substantially three-fourth the wavelength of the vibration λ generated by the vibrator 2, i.e. 3λ/4. The length of the vibrator 2 should be substantially one-half the wavelength of the vibrator (λ/2).

The suction mouth 4b formed at the leading end of the suction nozzle 4 is defined by cutting aslant the suction nozzle 4 at an angle of substantially 45° so as to direct toward the different direction from the axis X of the horn, as illustrated in Figure 3.

In the drawings, reference numeral 10 denotes a retaining member of elastic material for fixing elastically the rear end of the vibrator 2 onto the casing 1a, and 11 an electric power cord for supplying an alternating current to the vibrator 2.

In the illustrated embodiment as noted above, along the outer surface of the suction nozzle 4, there is provided a flushing pipe 12 so as to supply Ringer's solution or other cleaning fluid to around the suction mouth 4b formed in the leading end of the suction nozzle 4. The flushing pipe 12 is detachably retained by a joint portion 13a formed on the casing 1a and connected with a conduit 13b through which the aforementioned cleaning fluid is fed to the flushing pipe 12.

The ultrasonic surgical scalpel having the aforenoted structure according to this invention can be appropriately applied to a plastic surgical operation to remove subcutaneous adipose tissues as an example in the following manner.

First of all, the leading end of the suction nozzle 4 is inserted into under the skin of a patient's body, and then, the vibrator 2 is actuated by being applied with an alternating current to vibrate the horn 3 so that the subcutaneous adipose tissues of the patient's body are shattered into its mushy state by vibrations which are generated by the vibrator 2 and amplified by the horn 3. Simultaneously, sucking force is applied to inside the horn 3 for sucking the shattered subcutaneous tissues in the suction passage 4a inside the suction nozzle 4.

When the subcutaneous tissues shattered into the mushy state are sucked out, the operator who holds the handpiece 1 in his hand can freely regulate the sucking force by directly opening or closing the suction regulating mouth 7 with his finger tip.

That is, when the suction regulating mouth 7 is completely closed with the finger tip, the sucking force generated by the aspirator is applied into the suction passage 4a in the suction nozzle 4 without loss, with the result that the internal pressure in the suction passage 4a becomes equal to the sucking force fed from the aspirator. Thus, the suction force at this time in the suction passage 4a becomes the largest. On the other hand, by selectively opening the suction regulating mouth 7 with the finger tip, the sucking force given into the suction passage 4a can be varied in proportion to the opening size of the suction regulating mouth 7. In this manner the sucking force suitable for sucking out the shattered tissues can be obtained any time.

By supplying the cleaning fluid such as Ringer's solution to around the suction mouth 4b through the flushing pipe 12 in performing a surgical operation, the shattered tissues can be effectively sucked out.

Also in this embodiment, the scalpel has the acute leading end, so that resistance produced when inserting the suction nozzle 4 into the subcutaneous or organic tissue becomes relatively small. Therefore, the suction nozzle 4 can be smoothly inserted and moved forward in the tissue with ease. Besides, since the suction mouth 4b is open aside in the state directed toward the direction different from that of the axis X of the horn 3 or suction nozzle 4, only tissue to be removed can be effectively sucked out through the suction passage 4a.

Though the suction mouth 4b is formed by cutting aslant the suction nozzle 4 in the aforementioned embodiment, it may be of course formed in various ways so as to satisfy the condition that it opens toward the direction different from that of the axis X of the suction nozzle 4. For instance, as illustrated in Figure 4, there may be formed a suction mouth 14b in the circumferential side wall of the leading end part of the suction nozzle 4. In this case, the leading end of the suction nozzle 4 may be covered with a slant ellipse plate so as to assume an acute shape.

If the resistance produced when advancing the scalpel in the tissue of the living body is disregarded, the leading end of the suction nozzle 4 may be formed perpendicularly to the axis X in a blind state, as illustrated in Figure 5. To improve the advancing efficiency of the suction nozzle 4 of Figure 5, which brings about relatively large resistance when advancing in the tissue of the living body, the leading end of the suction nozzle 4 may preferably be formed round as shown in Figure 6. The suction mouths 24b and 34b formed in the leading end parts 4a of the suction nozzles 4 of the embodiments shown in Figures 5 and 6 are open toward the direction different from that of the axis of the respective suction nozzles.

As is clear from the foregoing disclosure, the ultrasonic surgical scalpel according to this invention enjoys an advantage that the suction nozzle formed on the leading end part of the horn can be stably and smoothly inserted into and moved forward in subcutaneous or organic tissues of a living body without needlessly injuring other tissues such as blood vessels and nerve tissues in advancing the scalpel in under the skin or organic tissues. This effect can be accomplished by forming the suction mouth in the leading end part of the suction nozzle so as to open aside toward the direction different from that of the axis of the suction nozzle. Furthermore, the suction regulating mouth which is provided in the course of the suction pipe connected with the suction nozzle and simple in structure enables the sucking force applied to the suction passage in the suction nozzle to be easily controlled with the finger tip of an operator.

## Claims

1. An ultrasonic surgical scalpel for shattering and sucking out subcutaneous or organic tissues of a living body and comprising a handpiece (1); an ultrasonic vibrator (2) contained within said handpiece (1), which generates ultrasonic vibrations by being applied with an alternating current; and a horn (3) formed with a suction nozzle (4), said horn (3) is connected to said vibrator (2) and adapted to transmit the vibrations to its leading end, said suction nozzle (4) is provided at its leading end with a suction mouth (4b,14b,24b,34b) open in the direction different from the axial direction (X) of the horn (3) and a suction passage (4a) thereinside, characterized in that said handpiece (1) is provided with a suction regulating mouth (7) in the form of an aperture in the wall of said suction passage (4a) inside said horn (3), which aperture can be fully or partially covered, e.g. with a finger tip, or uncovered in order to regulate the suction pressure in said suction passage, and in that said horn (3) is supported by a supporting member (5) at a point (Ps) substantially one-fourth wavelength (λ/4) of the vibration generated by the vibrator (2) away from a connection point (Pa) at which said horn (3) and vibrator (2) are connected, with said suction regulating mouth (7) being disposed at said support point (Ps).

2. An ultrasonic surgical scalpel according to Claim 1, characterized by further comprising a flushing pipe (12) provided along said suction nozzle (4) so as to supply cleaning fluid or the like to around said suction mouth (4b,14b,24b,34b), and said suction mouth is oriented away from an outlet of said flushing pipe (12).

3. An ultrasonic surgical scalpel according to Claim 1 or Claim 2, characterized by further comprising a lid member (7a) capable of being detachably fitted in said suction regulating mouth (7).

4. An ultrasonic surgical scalpel according to any one of Claims 1 - 3, characterized in that said suction mouth (4b) is formed by cutting aslant said suction nozzle (4) to form said leading end of the suction nozzle (4).

5. An ultrasonic surgical scalpel according to any one of Claims 1 - 4, characterized in that said leading end of the suction nozzle (4) is covered with a slant ellipse plate in a blind state, and said suction nozzle (4) has a circumferential side surface in which said suction mouth (14b) is formed.

6. An ultrasonic surgical scalpel according to any one of Claims 1 - 5, characterized in that said leading end of the suction nozzle (4) is formed perpendicularly to its axis (X) in a blind state, and said suction nozzle (4) has a circumferential side surface in which said suction mouth (24b) is formed.

7. An ultrasonic surgical scalpel according to any one of Claims 1 - 6, characterized in that the leading end of said suction nozzle (4) is in a round shape, and said suction nozzle (4) has a circumferential side surface in which said suction mouth (34b) is formed.

8. An ultrasonic surgical scalpel according to any one of Claims 1 to 7, characterized in that said suction nozzle (4) has a length substantially three-fourth the wavelength (λ) of the vibration generated by said vibrator (2).

## Patentansprüche

1. Chirurgisches Ultraschallskalpell zum Zertrümmern und Heraussaugen von subkutanen oder organischen Geweben eines lebenden Körpers, mit einem Handstück (1); einem Ultraschallvibrator (2), der innerhalb des Handstücks (1) enthalten ist, der beim Beaufschlagen mit einem Wechselstrom Ultraschallschwingungen erzeugt; und einem Horn (3), das mit einer Saugdüse (4) ausgebildet ist, wobei das Horn (3) mit dem Vibrator (2) verbunden und geeignet ist, um die Schwingungen zum vorderen Ende zu übertragen, wobei die Saugdüse (4) an ihrem vorderen Ende mit einer Saugöffnung (4b, 14b, 24b, 34b) versehen ist, welche in einer Richtung offen ist, die unterschiedlich zur axialen Richtung (X) des Horns (3) und eines innen liegenden Saugkanals (4a) ist, dadurch gekennzeichnet, daß das Handstück (1) mit einer Saugregulierungsöffnung (7) in der Form einer Öffnung in der Wand des Saugkanals (4a) innerhalb des Horns (3) versehen ist, wobei die Öffnung beispielsweise mit einer Fingerspitze vollständig oder teilweise bedeckt oder abgedeckt werden kann, um den Saugdruck im Saugkanal zu regulieren, und daß das Horn (3) von einem Halteteil (5) an einem Punkt (Ps) gehaltert ist, der im wesentlichen ein viertel der Wellenlänge (λ/4) der vom Vibrator (2) erzeugten Schwingung von einem Verbindungspunkt (Pa) entfernt ist, an dem das Horn (3) und der Vibrator (2) verbunden sind, wobei die Saugregulierungsöffnung (7) am Haltepunkt (Ps) angeordnet ist.

2. Chirurgisches Ultraschallskalpell nach Anspruch 1, dadurch gekennzeichnet, daß es ferner eine Spülleitung (12) umfaßt, die entlang der Saugdüse (4) vorgesehen ist, um Reinigungsfluid oder ähnliches um die Saugöffnung (4b, 14b, 24b, 34b) herum zuzuführen, und daß die Saugöffnung von einem Auslaß der Spülleitung (12) weggerichtet ist.

3. Chirurgisches Ultraschallskalpell gemäß Anspruch 1 oder 2, dadurch gekennzeichnet, daß es ferner ein Deckelteil (7a) umfaßt, daß lösbar in der Saugregulierungsöffnung (7) einpaßbar ist.

4. Chirurgisches Ultraschallskalpell nach einem der Ansprüche 1 bis 3, dadurch gekennzeichnet, daß die Saugöffnung (4b) gebildet ist, indem die Saugdüse (4) schräg geschnitten ist, um das vordere Ende der Saugdüse (4) zu bilden.

5. Chirurgisches Ultraschallskalpell nach einem der Ansprüche 1 bis 4, dadurch gekennzeichnet, daß das vordere Ende der Saugdüse (4) mit einer schrägen elliptischen Platte in einem abgedeckten Zustand bedeckt ist, und daß die Saugdüse (4) eine seitliche Umfangsfläche aufweist, in der die Saugöffnung (14b) ausgebildet ist.

6. Chirurgisches Ultraschallskalpell nach einem der Ansprüche 1 bis 5, dadurch gekennzeichnet, daß das vordere Ende der Saugdüse (4) senkrecht zu ihrer Achse (X) in einem abgedeckten Zustand ausgebildet ist, und daß die Saugdüse (4) eine seitliche Umfangsfläche aufweist, in der die Saugöffnung (24b) ausgebildet ist.

7. Chirurgisches Ultraschallskalpell nach einem der Ansprüche 1 bis 6, dadurch gekennzeichnet, daß das vordere Ende der Saugdüse (4) eine runde Form hat, und daß die Saugdüse (4) eine seitliche Umfangsfläche aufweist, in der die Saugöffnung (34b) ausgebildet ist.

8. Chirurgisches Ultraschallskalpell nach einem der Ansprüche 1 bis 7, dadurch gekennzeichnet, daß die Saugdüse (4) eine Länge hat, die im wesentlichen dreiviertel der Wellenlänge (λ) der vom Vibrator (2) erzeugten Schwingung ist.

## Revendications

1. Scalpel ultrasonique pour la chirurgie destiné à réduire en fragments et à évacuer par aspiration des tissus sous-cutanés ou organiques d'un corps vivant et comprenant un manche (1) ; un vibrateur ultrasonore (2) logé dans ledit manche (1), qui produit des vibrations ultrasonores lorsqu'on lui applique un courant alternatif ; et un cornet (3) formé avec une buse d'aspiration (4), ledit cornet (3) étant relié audit vibrateur (2) et étant adapté à transmettre les vibrations vers son extrémité avant, ladite buse d'aspiration (4) étant munie, à son extrémité avant, d'un orifice d'aspiration (4b, 14b, 24b, 34b) qui s'ouvre dans une direction différente de la direction axiale (X) du cornet (3) et d'un passage d'aspiration (4a), caractérisé en ce que ledit manche (1) est muni d'un orifice de régulation d'aspiration (7) sous la forme d'une ouverture formée dans la paroi dudit passage d'aspiration (4a) à l'intérieur dudit cornet (3), cette ouverture pouvant être complètement ou partiellement recouverte, par exemple, avec le bout du doigt, ou découverte afin de régler la pression d'aspiration dans ledit passage d'aspiration, et en ce que ledit cornet (3) est supporté par un élément de support (5) en un point (Ps) situé sensiblement à un quart de la longueur d'onde (λ/4) de la vibration produite par le vibrateur (2) par rapport à un point de liaison (Pa) au niveau duquel ledit cornet (3) et ledit vibrateur (2) sont reliés, ledit orifice de régulation d'aspiration (7) étant disposé au niveau dudit point de support (Ps).

2. Scalpel ultrasonique pour la chirurgie selon la revendication 1, caractérisé en ce qu'il comprend en outre un tube de rinçage (12) placé le long de ladite buse d'aspiration (4) afin d'amener un fluide de lavage ou équivalent autour dudit orifice d'aspiration (4b, 14b, 24b, 34b), et en ce que ledit orifice d'aspiration est orienté en sens opposé à une sortie dudit tube de rinçage (12).

3. Scalpel ultrasonique pour la chirurgie selon la revendication 1 ou 2, caractérisé en ce qu'il comprend en outre un couvercle (7a) capable d'être fixé de façon amovible dans ledit orifice de régulation d'aspiration (7).

4. Scalpel ultrasonique pour la chirurgie selon l'une quelconque des revendications 1 à 3, caractérisé en ce que ledit orifice d'aspiration (4b) est formé en coupant en biais ladite buse d'aspiration (4) de manière à former ladite extrémité avant de la buse d'aspiration (4).

5. Scalpel ultrasonique pour la chirurgie selon l'une quelconque des revendications 1 à 4, caractérisé en ce que ladite extrémité avant de la buse d'aspiration (4) est couverte d'une plaque elliptique inclinée en aveugle, et en ce que ladite buse d'aspiration (4) a une surface latérale circonférentielle dans laquelle est formé ledit orifice d'aspiration (14b).

6. Scalpel ultrasonique pour la chirurgie selon l'une quelconque des revendications 1 à 5, caractérisé en ce que ladite extrémité avant de la buse d'aspiration (4) est formée perpendiculairement à son axe (X) en aveugle, et en ce que ladite buse d'aspiration (4) a une surface latérale circonférentielle dans laquelle est formé ledit orifice d'aspiration (24b).

7. Scalpel ultrasonique pour la chirurgie selon l'une quelconque des revendications 1 à 6, caractérisé en ce que l'extrémité avant de ladite buse d'aspiration (4) a une forme arrondie, et en ce que ladite buse d'aspiration (4) a une surface latérale circonférentielle dans laquelle est formé ledit orifice d'aspiration (34b).

8. Scalpel ultrasonique pour la chirurgie selon l'une quelconque des revendications 1 à 7, caractérisé en ce que ladite buse d'aspiration (4) a une longueur sensiblement égale à trois quarts de la longueur d'onde (λ) de la vibration produite par ledit vibrateur (2).
